# EUROPEAN PATENT APPLICATION

(11) **EP 4 546 366 A1**
(43) Date of publication of application: **30.04.2025**
(21) Application number: 23205670.5
(22) Date of filing: 25.10.2023
(51) Int. Cl.: G16H 40/67, G16H 80/00

(54) **AUTOMATED CONTROL OF AUDIO STREAMS DURING REMOTE MEDICAL IMAGING SYSTEM CONTROL**

(71) Applicant: Koninklijke Philips N.V., 5656 AG Eindhoven (NL)
(72) Inventor: AMTHOR, Thomas Erik, Eindhoven (NL)
(74) Representative: Philips Intellectual Property & Standards

(57) **Abstract**

Disclosed herein is a medical system (100) comprising a network interface (112) configured for connecting to multiple remote medical imaging systems (104, 106, 108). The medical system further comprises a user interface (114) configured for remote control of the multiple remote medical imaging systems and an audio interface (122). The execution of machine executable instructions (130) causes a computational system (110) to: receive (200) medical system specific data streams (132, 134, 136) from the multiple remote medical imaging systems via the network interface, receive (202) a determination (140) of a selected remote medical imaging system (136) in response to inputting the medical system specific data streams into a selector module (138); and render (204) the at least one audio stream of the selected remote medical imaging system using the audio interface.

## Description

### TECHNICAL FIELD

The invention relates to the remote control of medical imaging systems.

### BACKGROUND

Various types of medical imaging systems such as magnetic resonance imaging systems, computer tomography systems, or other tomographic medical imaging systems are complicated and difficult to operate. To assist operators and ensure efficient operation of medical imaging systems they may be monitored or controlled remotely. A remote operator may have remote desktop control or access to multiple remote medical imaging systems simultaneously. In addition to having remote desktop control there may be additional audio and/or video feeds which enable the remote operator to interact with local operators of the multiple remote medical imaging systems.

European patent application EP3726834A1 discloses a telepresence system and a telepresence method for teleradiology. The telepresence system comprises an acquisition system located at a radiological diagnostic site and comprising one or more acquisition devices, wherein each acquisition device is configured to acquire a respective video stream comprising video data acquired of a region of the diagnostic site at which the respective acquisition device is located, and wherein the acquisition system is configured to transmit the one or more video streams to a controller, a user interface system located remotely from the diagnostic site, wherein the user interface system comprises a user communication input unit and a replay unit with an immersive display, and a user communication output system located at the diagnostic site. The controller is configured to receive the one or more video streams and to transmit at least one of the video streams to the user interface system, wherein the replay unit is configured to output the at least one video stream on the immersive display to a user, and the user communication input unit is configured to receive a user input and generate a user input signal representative of the user input, wherein the user interface system is configured to transmit the user input signal to the controller, and the controller is configured to receive the user input signal and to transmit the user input signal to the user communication output system for output of the user input at the diagnostic site.

### SUMMARY

The invention provides for a medical system, a computer program, and a method in the independent claims. Embodiments are given in the dependent claims.

In one aspect the invention provides for a medical system that comprises a network interface that is configured for connecting to multiple remote medical imaging systems. The medical system further comprises a user interface that is configured for remote control of the multiple remote medical imaging systems. The medical system further comprises an audio interface.

The medical system further comprises a memory storing machine-executable instructions and a selector module. The medical system further comprises a computational system; the execution of the machine-executable instructions causes the computational system to receive medical system specific data streams from the multiple remote medical imaging systems via the network interface. The medical system specific data streams individually comprise at least one audio stream and user interface control data. The selector module is configured to determine a selected remote medical imaging system from the multiple remote medical imaging systems in response to receiving the medical system specific data streams.

Execution of the machine-executable instructions further causes the computational system to receive the determination of the selected remote medical imaging system in response to inputting the medical system specific data streams into the selector module. Execution of the machine-executable instructions further causes the computational system to render the at least one audio stream of the selected remote medical imaging system using the audio interface. The at least one audio stream of the multiple remote medical imaging systems are selected by the selector module or muted or rendered with a reduced volume using the audio interface.

In another aspect, the invention provides for a computer program that comprises machine-executable instructions for execution by a computational system configured for controlling a medical system. The machine-executable instructions further comprise a selector module for execution by the computational system. The medical system comprises a network interface configured for connecting to multiple remote medical imaging systems. The medical system further comprises a user interface that is configured for remote control of the multiple remote medical imaging systems. The medical system further comprises an audio interface.

Execution of the machine-executable instructions causes the computational system to receive medical system specific data streams from the multiple remote medical imaging systems via the network interface. The medical system specific data streams individually comprise at least one audio stream and remote user interface control data. The selector module is configured to choose a selected remote medical imaging system from the multiple remote medical imaging systems in response to receiving the medical system specific data streams.

Execution of the machine-executable instructions further causes the computational system to receive the determination of the selected remote medical imaging system in response to inputting the medical system specific data streams into the selector module. Execution of the machine-executable instructions further causes the computational system to render the at least one audio stream of the selected remote medical imaging system using the audio interface. The at least one audio stream of the remote medical imaging systems not selected by the selector module are muted or rendered with a reduced volume using the audio interface.

In another aspect the invention provides for a method of operating a medical system. The medical system comprises a network interface configured for connecting to multiple remote medical imaging systems. The medical system further comprises a user interface configured for remote control of the multiple remote medical imaging systems. The medical system further comprises an audio interface.

The method comprises receiving the medical system specific data streams from the multiple remote medical imaging systems via the network interface. The medical system specific data streams individually comprise at least one audio stream and remote user interface control data. The method further comprises receiving the determination of the selected remote medical imaging system in response to inputting the medical system specific data streams into a selector module. The selector module is configured to choose a selected remote medical imaging system from the multiple remote medical imaging systems in response to receiving the medical system specific data streams. The method further comprises rendering the at least one audio stream of the selected remote medical imaging system using the audio interface. The at least one audio stream of the multiple remote medical imaging systems not selected by the selector module at muted or are rendered with a reduced volume using the audio interface.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 illustrates an example of a focus indicator.
Fig. 5 illustrates a further example of a focus indicator.

### DESCRIPTION OF EMBODIMENTS

Like numbered elements in these figures are either equivalent elements or perform the same function. Elements which have been discussed previously will not necessarily be discussed in later figures if the function is equivalent.

In one example a medical system comprises a network interface configured for connecting to multiple remote medical imaging systems. The network interface may for example be a local area network or internet connection configured for connecting the multiple remote medical imaging systems to the medical system. The multiple remote medical imaging systems may be various types of medical imaging systems such as a magnetic resonance imaging system, a computed tomography system, a single-photon emission tomography system, a positron emission tomography system, or an imageguided radiotherapy system. The remote medical imaging systems may for example have a computer or user interface which is used to locally control that medical imaging system. The network interface may connect to this computer or computing system which is used locally for the control of a particular medical imaging system.

The medical system further comprises a user interface configured for remote control of the multiple remote medical imaging systems. This may for example comprise one or more human interface devices such as a monitor, touchscreen, or mouse which enables the control of the multiple remote medical imaging systems. Often times the remote medical imaging systems may be legacy systems or systems constructed by other vendors. As such, there may or may not be access to the software code for the remote medical imaging system. However, it is possible to control a remote medical imaging system remotely by providing a rendering of the local user interface for a remote medical imaging system as well as providing remote access to a mouse or other human interface device control. The user interface, which is local to the medical system, may therefore be configured to remotely control the user interface of the multiple remote medical imaging systems.

The medical system further comprises an audio interface. The audio interface may provide capabilities for rendering an audio stream as well as a microphone for recording sounds from an operator using the medical system. The medical system further comprises a memory storing machine-executable instructions and a selector module. The selector module may be executable code and may be configured to choose a selected remote medical imaging system from the remote medical imaging systems in response to receiving medical system specific data streams.

In various examples, the medical system specific data streams may include, but are not limited to: one or multiple video streams for console screen sharing, to allow the remote expert to view of one or multiple console displays; optional video streams to transmit camera images of any number of cameras installed in the imaging system control, preparation, or exam rooms; video streams (both directions) as part of a video call between the local and remote staff; audio streams (both directions) as part of an audio or video call between the local and remote staff; optional audio streams (both directions) to facilitate communication between the patient in the exam room and the remote expert; data stream for transmitting console control commands from the remote expert to the imaging system; optional data stream of system state information to transmit operational details, such as elapsed exam time, exam progress, protocol and patient details; and combinations thereof.

Execution of the machine-executable instructions causes the computational system to receive medical system specific data streams from the multiple remote medical imaging systems via the network interface. This step may be performed repeatedly. The medical system specific data streams individually comprise at least one audio stream and remote user interface control data. The remote user interface control data may be the data which enables control of the local user interface of a particular remote medical imaging system. This may for example be a rendering of a monitor or visual display as well as providing remote access to a mouse control. Individual remote medical imaging systems provide a medical system specific data stream. These comprise the remote user interface control data that was mentioned above as well as at least one audio stream. The at least one audio stream may comprise audio from an operator or other microphones which are located at the facility of the particular remote medical imaging system.

Execution of the machine-executable instructions further causes the computational system to receive the choice of the selected remote medical imaging system in response to inputting the medical system specific data stream into the selector module. This step may be performed repeatedly. The multiple remote medical imaging systems may individually provide the medical system specific data streams and these data streams are then input into the selector module. From this data, the selector module then chooses a selected remote medical imaging system. Execution of the machine-executable instructions further causes the computational system to render the at least one audio stream of the selected remote medical imaging system using the audio interface. This step may be performed repeatedly. The at least one audio stream of the multiple remote medical imaging systems not selected by the selector module are muted or are rendered with a reduced volume using the audio interface.

The muting of the at least one audio stream of the multiple remote medical imaging systems which are not selected may also cover interrupting the audio signal.

Examples may be beneficial because they may provide for a means of automatically rendering the audio from a selected remote medical imaging system. The medical system may for example be used for controlling a large number of remote medical imaging systems. It may be difficult or impossible for a human operator to choose which of the multiple remote medical imaging systems should have its audio emphasized.

In one example the multiple remote medical imaging systems is a single remote medical imaging system. In principle it could be possible to have more than one audio stream from a single system, for example one audio stream to the local operator and one to the patient in the exam room. In this case, the selector module could even choose from or selectively attenuate the individual audio streams from the 1 system. The current medical systems have only a single audio channel per system. Multiple audio streams from a single system or single medical system specific data stream is also possible.

When interacting with or supervising more than one remote medical imaging system, the remote expert or operator of the medical system may be aware of what is happening in the different control rooms of the multiple remote medical imaging systems. It may be that in many cases the audio stream to more than one remote medical imaging system control room are active simultaneously. This may have the following disadvantages:
1. The remote expert cannot always judge who the person currently speaking is, and where this person is located.
2. While intending to speak to one specific connected local technologist, other connected technologists from different sites may hear what the remote expert says. This can lead to confusion about the imaging procedure and bears the risk of unintentionally revealing sensitive patient information to the wrong recipient.
3. When important audible information requires an immediate reaction, such as an alarm signal triggered by a patient's alarm button, it may not be clear from which site the signal was received.

Various examples may potentially overcome these problems by:
1. Displaying information about the source of the currently audible voice for the remote expert and
2. Selectively attenuate audio signals from/to individual connected imaging system control rooms depending on context and user focus using the selector module.
3. Detecting important patterns in the audio streams, such as alarm signals, and providing the remote expert with respective localization information.

In another example the audio interface further comprises a microphone configured for receiving a user audio signal. This for example may be an audio signal recorded or digitized from the user or operator of the medical system. Execution of the machine-executable instructions further causes the computational system to transmit the user audio signal to the selected remote medical imaging system exclusively. The selector module is used to select which of the multiple remote medical imaging systems provides an audio signal to the operator of the medical system. This example may be beneficial because it automatically configures the medical system such that the audio provided by the operator of the medical system is only provided to the selected remote medical imaging system. This for example prevents a confusing situation where the operator of the medical system and the different multiple remote medical imaging systems are unaware if the audio is directed towards them or not. This automatic configuration may eliminate or reduce the need to continually configure the audio interface manually.

In some instances, this example may also provide audio from other of the multiple remote medical imaging systems being provided to each other unintentionally. It may for example provide for a better level of privacy as well as reducing the amount of unnecessary noise and chatter which may be distracting to an operator of a remote medical imaging system.

In another example the selector module comprises an audio pattern detection module that is configured for detecting an audio pattern in the at least one audio stream of the multiple remote medical imaging systems. The selector module is configured to choose the selected remote medical imaging system by detecting the audio pattern. This example may be beneficial because the detection of a particular audio pattern may be useful in detecting if there is an alarm or alert situation at a particular remote medical imaging system. If this is detected then the system can automatically configure the audio exchange between the chosen or selected remote medical imaging system and the audio interface of the medical system. In some examples, the audio pattern may be a predetermined audio pattern.

The audio pattern detection module may function differently in different examples. In one case the audio pattern detection module could for example do a Fourier transform of the incoming audio streams and then detect an elevated presence of a particular frequency. This may for example be useful in detecting the presence of an alarm. In other examples various neural networks or machine learning modules could be trained to detect the audio pattern.

In another example the audio pattern is an alarm signal. This embodiment may be beneficial because it may be used to automatically detect a fault or alarm situation at the site of the selected remote medical imaging system. This may enable the audio exchange to be established between the selected remote medical imaging system and the medical system audio interface automatically.

In another example, the selector module comprises an audio-to-speech converter module configured to output a medical system specific text stream. The selector module is configured to generate the medical system specific text stream in response to receiving the at least one audio stream of the multiple remote medical imaging systems using the speech converter module. The selector module is further configured to choose the medical imaging system as the selected remote medical imaging system for which a predetermined word or phrase is detected in the medical imaging system specific text stream. This embodiment may be beneficial because it may use the words of an operator of the selected remote medical imaging system to initiate the audio configuration. This for example may be used to establish the audio link when words which indicate that the operator of the selected remote medical imaging system is having trouble or specifically requests a connection for the audio connection to be made.

The audio-to-speech converter module may for example be constructed using standard audio-to-speech converters such as is used for dictation software or is even currently available in such things as smartphones and office productivity software.

In some examples the text screen can be compared to a template.

In another example, the remote user interface control data comprises image data. The selector module comprises a pattern recognition module configured to choose the selected remote medical imaging system using the image data of the remote medical imaging systems. As was mentioned above, the remote user interface control data may comprise data such as the rendering of a user interface at the local site of the different remote medical imaging systems. The selector module may comprise a pattern recognition module that is able to look at these images and then recognize specific words, text, symbols or other data which may trigger the selector module to choose the selected remote medical imaging system. This for example may be beneficial because the medical system may then be able to recognize various symbols or alerts or warnings on a remote user interface.

In another example, the pattern recognition module is an image classification neural network configured to output the choice of the selected remote medical imaging system in response to receiving the image data of the multiple remote medical imaging systems as input. For example, this may be implemented using a convolutional neural network such as is commonly used for image classification. The pattern recognition module may be trained by taking or collecting a combination of user interface renderings over a period of time and then manually labeling them with the choice of the selected remote medical imaging system. They may for example be trained using a deep learning procedure.

In another example, the remote user interface control data comprises text data. The selector module comprises a text recognition neural network that is configured to output the choice of the selected remote medical imaging system in response to receiving the text data of the multiple remote medical imaging systems as input. This example may be beneficial because it is very flexible to program a text recognition neural network to have a complex response to text data. This may be a means of providing for exceptional selection of the selected remote medical imaging system. The text recognition neural network may for example a convolutional neural network that is configured for identifying particular words or phrases. In another example the text recognition neural network is a recurrent neural network. In another example the text recognition neural network is a large language model.

A large language model (LLM) as used herein encompasses a neural network architecture, typically built up of transformers (encoders and decoders) with self-attention layers and residual connections, that is a language model that has been trained using unlabeled text using selfsupervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

In this particular example, the LLM may be trained by collection exemplary text data and pairing it with feedback generated by a human and training it with RLHF.

In another example the user interface is configured to receive operator control data configured to control one of the remote medical imaging systems. The selector module is further configured to receive the operator control data from the user interface. The selector module is further configured to use the one of the remote medical imaging systems as a choice of the selected remote medical imaging system.

This example may be beneficial because as the operator of the medical system is controlling a particular remote medical imaging system, it may automatically gain focus such that the operator of the medical system is able to communicate with the operator of the selected remote medical imaging system via audio automatically.

In another example the medical system further comprises an image viewing determination device configured to determine a currently viewed remote medical imaging system played by the user interface belonging to the multiple remote medical imaging systems. The selector module is further configured to use the currently viewed remote medical imaging system as the choice of the selected remote medical imaging system. This example may be beneficial because it automatically shifts the audio connection to the remote medical imaging system which is being viewed on the user interface. In some examples, the image viewing determination device could be an eye tracking system that determines where the user's eyes are looking.

In another example, the user interface is configured to display a focus indicator to indicate the selected remote medical imaging system. The focus indicator may for example be an icon or other optical indicator such as an increased brightness or an indicator surrounding the user interface of the selected remote medical imaging system on the user interface. This may be beneficial because it may help reduce the chances that the operator of the medical system is confused as to which is the selected remote medical imaging system.

In another example the medical system further comprises the multiple remote medical imaging systems.

In another example the remote medical imaging system comprises a magnetic resonance imaging system.

In another embodiment the multiple remote medical imaging systems comprises a positron emission tomography system.

In another embodiment the multiple remote medical imaging systems comprises a single photon emission tomography system.

In another embodiment the multiple remote medical imaging systems comprises a fluoroscope.

In another embodiment the multiple remote medical imaging systems comprises an ultrasound system.

In another embodiment the multiple remote medical imaging systems further comprises a magnetic resonance imaging-guided radiotherapy system.

In another embodiment the multiple remote medical imaging systems comprises a computed tomography-guided radiotherapy system.

In another embodiment the multiple remote medical imaging systems comprises a computed tomography system.

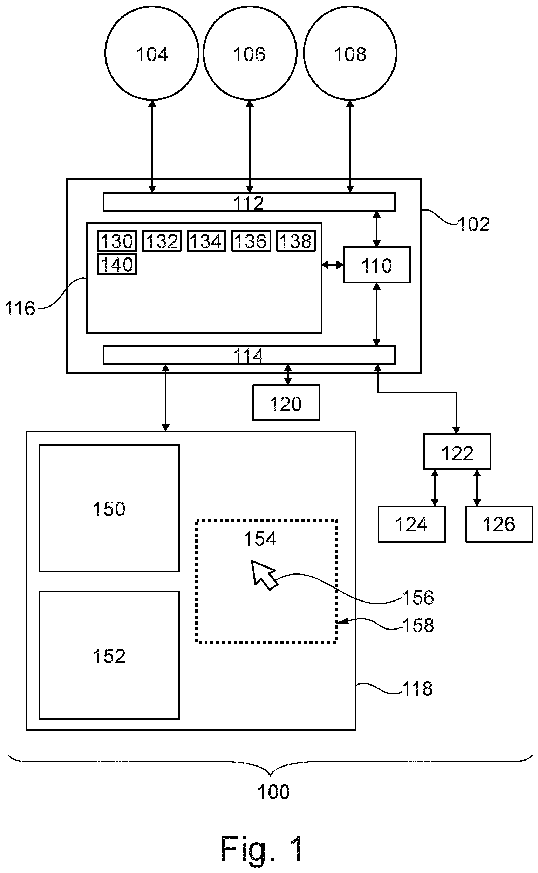

Fig. 1 illustrates an example of a medical system 100. The medical system 100 is shown as comprising a computer 102. Additionally shown is a first remote medical imaging system 104, a second remote medical imaging system 106 and a third remote medical imaging system 108. These multiple remote medical imaging systems 104, 106, 108 in some examples will be part of the medical system 100 and in other examples will be separate from it.

The computer 102 is shown as comprising a computational system 110. The computational system 110 is intended to represent one or more computational systems or computing cores. The computer 102 is further shown as comprising a network interface 112. The network interface 112 forms a network interface or connection with the first remote medical imaging system 104, the second remote medical imaging system 106, and the third remote medical imaging system 108. The computational system 110 is shown as being in communication with the network interface 112. The computational system 110 is in further communication with a user interface 114. The computational system 110 is in further communication with a memory 116. The memory 116 is intended to represent various types of memory that are accessible to the computational system 110. The user interface 114 is shown as having a number of sub-components. The user interface 114 comprises a graphical user interface 118. The user interface 114 is shown as optionally comprising an image viewing determination device 120. The user interface 114 is further shown as comprising an audio interface 122 with a speaker 124 and a microphone 126.

The memory is shown as comprising machine-executable instructions 130. The machine-executable instructions 130 enable the computational system 110 to control the medical system 100 as well as perform various numerical and data processing tasks. The memory 116 is further shown as comprising a medical-specific data stream 132 from the first remote medical imaging system 104. The memory 116 is further shown as comprising or storing a medical system specific data stream 134 from the second remote medical imaging system 106. The memory 116 is further shown as storing a medical-specific data stream 136 from the third remote medical imaging system 108.

The memory 116 is further shown as comprising a selector module 138. The selector module 138 receives the medical system specific data streams 132, 134, 136 as input and outputs a determination 140 of a selected remote medical imaging system. Determination can be called choice interchangeably in the present application for simplicity reasons. In this example the selected remote medical imaging system is the third remote medical imaging system 108. The graphical user interface 118 is shown as comprising a number of items which are displayed. The graphical user interface shows a rendering 150 of the user interface of the first remote medical imaging system 104. The graphical user interface 118 is further shown as showing a rendering 152 of the user interface of the second remote medical imaging system 106. The graphical user interface 118 is further shown as comprising or displaying a rendering 154 of the user interface of the third remote medical imaging system 108. This for example could be screens which are displayed on the graphical user interface 118 and provide the user interface to the various remote medical imaging systems 104, 106, 108. The third remote medical imaging system 108 is the selected remote medical imaging system and its rendering 154 shows a focus indicator 158. The focus indicator 158 indicates that there is an audio connection established between the third remote medical imaging system 108 and the audio interface 122.

The selection of the selected remote medical imaging system 108 could be achieved in several ways. In one example the medical system specific data streams 132, 134, 136 are input into the selector module 138 and the choice 140 is received. The graphical user interface 118 is shown as having a mouse pointer 156 over the rendering 154 of the user interface of the third remote medical imaging system 108. The use or the positioning of the mouse pointer 156 could also be used to select the selected remote medical imaging system 108. In other examples the image viewing determination device 120 could be used when the operator is looking at the rendering 154 of the user interface of the third remote medical imaging system 108 the third remote medical imaging system is selected.

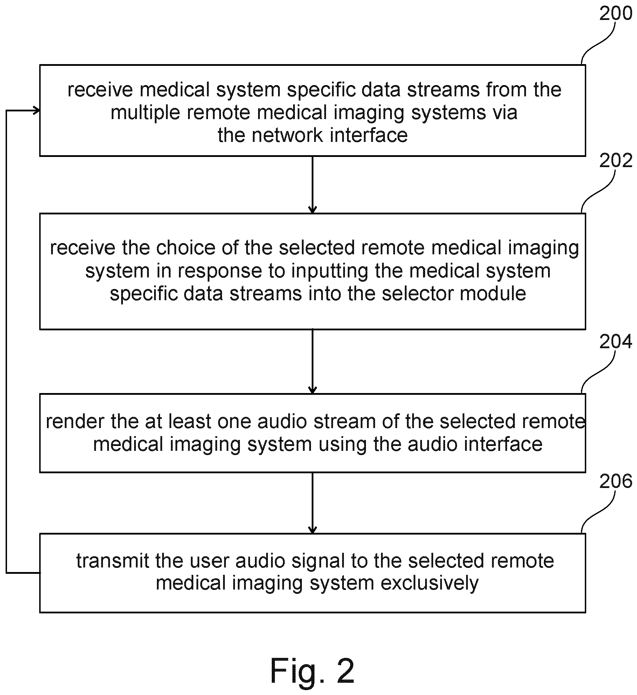

Fig. 2 shows a flowchart which illustrates a method of operating the medical system 100 of Fig. 1. In step 200 the medical system specific data streams 132, 134, 136 are received from the multiple remote medical imaging systems 104, 106, 108 via the network interface 112. In step 202, the choice of the selected remote medical imaging system 108 is received in response to inputting the medical system specific data streams 132, 134, 136 into the selector module 138. In step 204, the at least one audio stream of the selected remote medical imaging system 108 is rendered using the audio interface 122. Step 206 is optional. In step 206, the user audio signal is transmitted to the selected remote medical imaging system 108 exclusively. The steps 200, 202, 204, and 206 may be repeated in a loop so that as the medical system 100 is used the system which receives the focus and the exchange of the audio signals may change as operating conditions of the medical system 100 also change.

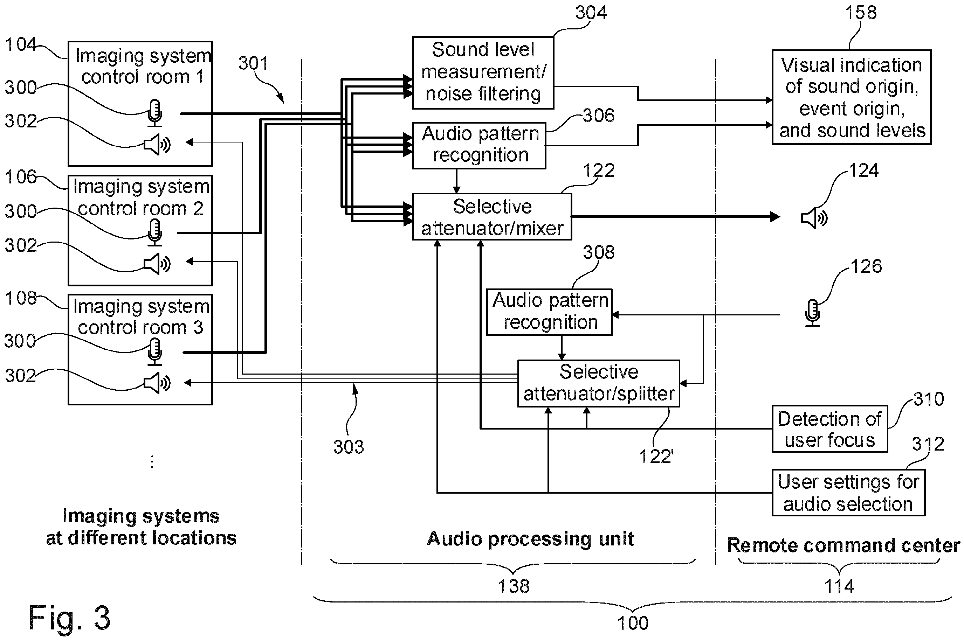

Fig. 3 illustrates one alternative implementation of the medical system 100 illustrated in Fig. 1. The remote medical imaging systems 104, 106, 108 are each shown as comprising a remote microphone 300 and a remote speaker 302. The remote microphone 300 provides an audio stream 301 which is sent over the network. The remote speakers 302 are used to render a user audio signal 303 provided by the microphone 126. The selector module 138 is shown as having a number of components which in this case are combined with the functionality of the audio interface 122, 122'. There is a sound level measurement and possibly noise filtering module 304 which is used to detect large amounts of noise or particular noises from the remote microphones 300. The audio may also be fed to an audio pattern recognitions module 306. These may be used to provide visual indications of sound, event origins, and sound levels in box 158.

The audio pattern recognition module 306 may for example function as the selector module 138 and may control a selective attenuator or mixer 122 which mixes or controls the audio stream 301 from the various remote microphones 300. The user interface 114 functions as a remote command center and may provide this visual indication 158. It may also provide the speaker 124 and the microphone 126. There may also be functionality for the detection of the user focus 310, for example, using the optional image viewing determination device 120 or the detection of the mouse pointer 156. The user interface 114 may also comprise a means for user settings for audio selection 312. For example the system could be programmed to override the selector module 138. A portion of the selector module 138 may be an audio pattern recognition module 308 which also monitors the microphone 126. For example, the operator of the medical system 100 could say which imaging system 104, 106, 108 to give focus to. The audio system 122 is also shown as comprising a selective attenuator or splitter 122' which functions as a mixer for controlling the user audio signal 303 fed to each of the remote speakers 302. In many cases the functionality of the mixer 122 and the mixer 122' have their functionality linked. For example, if the signal from the remote microphone 300 of the first remote imaging system 104 is given focus then the mixer 122' may actively cut off the user audio signal 303 to the other remote medical imaging systems 106, 108.

The selector module 138 in the above example is implemented as an audio processing unit. This selector module 138 can be implemented in software and run on a computer located physically in the remote command center (e.g., as part of the computing system), or on the cloud computing system. The audio processing unit consists of several modules managing the audio streams in both directions, in particular including logic to selectively attenuate individual audio inputs and outputs and the detection of audio patterns.

The Sound level measuring and noise filter module 304 may determine the volume of sound / speech in each imaging control room. A noise filter may be applied to suppress background noise. The purpose of this module is to determine which local operator is currently speaking into the microphone. The sound levels measured from each incoming audio stream are reported to the visualization module.

The Audio pattern recognition module 308 may employ audio recognition technologies (e.g., based on Fourier analysis to detect certain frequency patterns or based on AI sound recognition) to detect important audible events. A typical example would be to detect the alarm signal sounding in an operating room when the patient presses the alarm button in an MRI.

The information about detected audio patterns and their origins is forwarded to the visualization module and also serves as a control input to the selective attenuator/mixer module to be able to automatically focus on the audio stream of a site where a critical audio signal has been detected.

The selective attenuator/mixer module 122 may mix the incoming audio signals using a weighted sum (with weights for example between 0 and 1). The weights for the individual inputs determine which audio signal is attenuated. They are determined from control inputs connected to the audio pattern recognition module, the user focus detection, and the user settings module.

The audio pattern recognition module 308 may employs audio recognition technologies (e.g., based on AI sound recognition) to detect important keywords or sentence structures in the remote operator's speech. For example, modern AI technology, such as GPT-4, can be used to determine the context of a sentence. If a word, expression, or sentence contains sensitive information or information that is otherwise restricted to the communication with one particular site, the module can control the selective splitter/mixer module in such a way that the audio signal is only transmitted to its intended recipient and will be attenuated for all other recipients. This may require buffering the audio signal for a given period of time (e.g., 2 seconds) to make sure that no information has already been transmitted before the context of the sentence could be identified.

The selective splitter/mixer module 122' may split the audio signal originating from the command center to multiple outputs connected to the imaging system control rooms, using individual weights for attenuation (with weights for example between 0 and 1). The weights are determined from control inputs connected to the audio pattern recognition module, the user focus detection, and the user settings module.

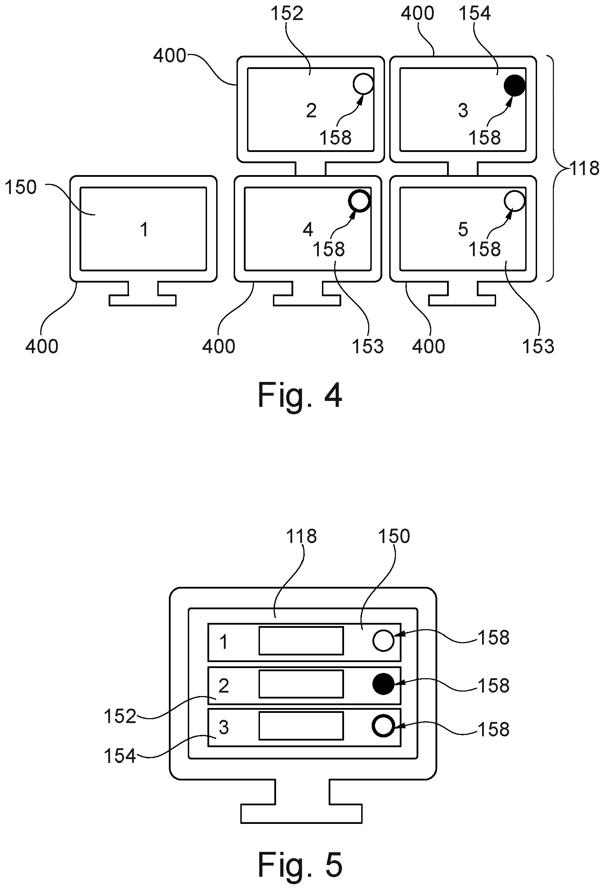

Figs. 4 and 5 illustrate several examples of focus indicators 158. The expert user at the command center desk of a medical system 100 typically has a screen setup similar to the ones illustrated in Figs. 4 and 5, where information from multiple connections us shown either on individual screens (eg., console screen sharing) or as a summary view on a single screen.

In Fig. 4, the graphical user interface 118 is formed from a collection of discrete screens 400. Each screen shows a rendering of a remote user interface 150, 152, 153, 154, 155 from a different remote medical imaging system. In Fig. 4, the graphical user interface 118 additionally displays a rendering of a fourth remote medical imaging system 153 and a rendering of a fifth remote medical imaging system 155. In some examples one of the screens, may be a control interface for the operator or user of the medical system. In Fig. 4, screen 150 does not show a focus indicator 158 and could be interpreted as being a control interface for the medical system 100.

Visual indicators of the origin of a voice or other sound currently being heard can be realized by displaying icons (focus indicators 158) of different intensity (color or brightness) on the respective screens 400 or UI elements. In Fig. 4, the focus indicators 158 are circles, where the intensity of the fill color represents the audio level from the remote medical imaging system 104, 106, 108. A darker fill color means that a larger audio signal originates from the respective imaging system console. In this way, when hearing the voice of one of the local operators, the remote expert can immediately identify the origin of the voice and the respective connected imaging system.

In Fig. 5, the rendering of the system data stream from the remote medical imaging system 104, 106, and 108 is shown on a single screen graphical user interface 118. The focus indicators 158 in Fig. 5 are implemented in the same way as was implemented in Fig. 4.

In other examples, the focus indicator 158 can also be realized with other symbols, external LEDs, borders around the image on the screen, etc.

Audible events identified by the selector module 138 such as alarm bells, can also be represented by visual indicators on the screen. These events can be illustrated by visualizing additional icons, possibly flashing, additional LEDs, or any variation of the audio level indicators described above.

In order to improve user experience even more, the selective mixing 122 and splitting modules 122' can be dynamically configured based on user focus. In this way, the remote expert will be able to communicate more efficiently with individual imaging system operators, while still keeping multiple audio connections active simultaneously.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: first remote medical imaging system
- 106: second remote medical imaging system
- 108: third remote medical imaging system
- 110: computational system
- 112: network interface
- 114: user interface
- 116: memory
- 118: graphical user interface
- 120: image viewing determination device
- 122: audio interface
- 124: speaker
- 126: microphone
- 130: machine executable instructions
- 132: medical system specific data stream from first remote medical imaging system
- 134: medical system specific data stream from second remote medical imaging system
- 136: medical system specific data stream from third remote medical imaging system
- 138: selector module
- 140: choice of the selected remote medical imaging system
- 150: rendering of user interface of first remote medical imaging system
- 152: rendering of user interface of second remote medical imaging system
- 153: rendering of user interface of fourth remote medical imaging system
- 154: rendering of user interface of third remote medical imaging system
- 155: rendering of user interface of fifth remote medical imaging system
- 156: mouse pointer
- 158: focus indicator
- 200: receive medical system specific data streams from the multiple remote medical imaging systems via the network interface
- 202: receive the choice of the selected remote medical imaging system in response to inputting the medical system specific data streams into the selector module
- 204: render the at least one audio stream of the selected remote medical imaging system using the audio interface
- 206: transmit the user audio signal to the selected remote medical imaging system exclusively
- 300: remote microphone
- 301: audio stream
- 302: remote speaker
- 303: user audio signal
- 304: sound level measuring / noise filtering
- 306: audio pattern recognition
- 308: audio pattern recognition
- 310: detection of user focus
- 312: user settings for audio selection
- 400: discrete screen

## Claims

1. A medical system (100) comprising:
- a network interface (112) configured for connecting to multiple remote medical imaging systems (104, 106, 108);
- a user interface (114) configured for remote control of the multiple remote medical imaging systems;
- an audio interface (122);
- a memory (116) storing machine executable instructions (130) and a selector module (138);
- a computational system (110), wherein execution of the machine executable instructions causes the computational system to:
- receive (200) medical system specific data streams (132, 134, 136) from the multiple remote medical imaging systems via the network interface, wherein the medical system specific data streams individually comprise at least one audio stream (301) and remote user interface control data, wherein the selector module is configured to determine a selected remote medical imaging system from the multiple remote medical imaging systems in response to receiving the medical system specific data streams;
- receive (202) a determination (140) of the selected remote medical imaging system in response to inputting the medical system specific data streams into the selector module; and
- render (204) the at least one audio stream of the selected remote medical imaging system using the audio interface, wherein the at least one audio stream of the multiple remote medical imaging systems not selected by the selector module are muted or are rendered with a reduced volume using the audio interface.

2. The medical system of claim 1, wherein the audio interface further comprises a microphone (126) configured for receiving a user audio signal (303), wherein execution of the machine executable instructions further causes the computational system to transmit (206) the user audio signal to the selected remote medical imaging system exclusively.

3. The medical system of claim 1 or 2, wherein the selector module comprises an audio pattern detection module (306) configured for detecting an audio pattern in the at least one audio stream of the multiple remote medical imaging systems, wherein the selector module is configured to choose the selected remote medical imaging system by detecting the audio pattern.

4. The medical system of claim 3, wherein the audio pattern is a signal of interest, such as an alarm signal.

5. The medical system of any one of the preceding claims, wherein the selector module comprises an audio to speech converter module configured to output a medical system specific text stream, wherein the selector module is configured to:
- generate the medical system specific text stream in response to receiving the at least one audio stream of the multiple remote medical imaging systems using the speech converter module;
- choose the medical imaging system as the selected remote medical imaging system for which a predetermined word or phrase is detected in the medical imaging system specific text stream.

6. The medical system of any one of the preceding claims, wherein the remote user interface control data comprises image data, wherein the selector module comprises a pattern recognition module configured to choose the selected remote medical imaging system using the image data of the multiple remote medical imaging systems.

7. The medical system of claim 6, wherein the pattern recognition module is an image classification neural network configured to output the choice of the selected remote medical imaging system in response to receiving the image data of the multiple remote medical imaging systems as input.

8. The medical system of 6 or 7, wherein the remote user interface control data comprises text data, wherein the selector module comprises a text recognition neural network configured to output the choice of the selected remote medical imaging system in response to receiving the text data of the multiple remote medical imaging systems as input.

9. The medical system of any one of the preceding claims, wherein the user interface is configured to receive operator control data configured to control one of the remote medical imaging systems, wherein the selector module is further configured to receive the operator control data from the user interface, and wherein the selector module is further configured to use the one of the remote medical imaging systems as the choice of the selected remote medical imaging system.

10. The medical system of any one of the preceding claims, wherein the medical system further comprises an image viewing determination device (120) configured to determine a currently viewed remote medical imaging system displayed by the user interface belonging to the multiple remote medical imaging systems, wherein the selector module is further configured to use the currently viewed remote medical imaging system as the choice of the selected remote medical imaging system.

11. The medical system of any one of the preceding claims, wherein the user interface is configured to display a focus indicator to indicate the selected remote medical imaging system.

12. The medical system of any one of the preceding claims, wherein the medical system further comprises the multiple remote medical imaging systems.

13. The medical system of any one of the preceding claims, wherein the multiple remote medical imaging systems comprise any one of the following: a magnetic resonance imaging system, a positron emission tomography system, a single photon emission tomography system, a digital fluoroscope, an ultrasound system, a magnetic resonance imaging guided radiotherapy system, a computed tomography guided radiotherapy system, a computed tomography system, and any combinations thereof.

14. A computer program comprising machine executable instructions (130) for execution by a computational system configured for controlling a medical system, wherein the computer program further comprises a selector module (138) for execution by the computational system, wherein the medical system comprises a network interface (112) configured for connecting to multiple remote medical imaging systems (104, 106, 108), wherein the medical system further comprises a user interface (114) configured for remote control of the multiple remote medical imaging systems; wherein the medical system further comprises an audio interface (122), wherein execution of the machine executable instructions causes the computational system to:
- receive (200) medical system specific data streams (132, 134, 136) from the multiple remote medical imaging systems via the network interface, wherein the medical system specific data streams individually comprise at least one audio stream (301) and remote user interface control data, wherein the selector module is configured to determine a selected remote medical imaging system from the multiple remote medical imaging systems in response to receiving the medical system specific data streams;
- receive (202) a determination (140) of the selected remote medical imaging system in response to inputting the medical system specific data streams into the selector module; and
- render (204) the at least one audio stream of the selected remote medical imaging system using the audio interface, wherein the at least one audio stream of the multiple remote medical imaging systems not selected by the selector module are muted or are rendered with a reduced volume using the audio interface.

15. A method of operating a medical system (100), wherein the method comprises:
- receiving (200) medical system specific data streams (132, 134, 136) from the multiple remote medical imaging systems via the network interface, wherein the medical system specific data streams individually comprise at least one audio stream (301) and remote user interface control data;
- receiving (202) a determination (140) of a selected remote medical imaging system in response to inputting the medical system specific data streams into a selector module, wherein the selector module is configured to choose the selected remote medical imaging system from the multiple remote medical imaging systems in response to receiving the medical system specific data streams;
- rendering (204) the at least one audio stream of the selected remote medical imaging system using the audio interface, wherein the at least one audio stream of the multiple remote medical imaging systems not selected by the selector module are muted or are rendered with a reduced volume using the audio interface.
